# EUROPEAN PATENT APPLICATION

(11) **EP 4 582 057 A2**
(43) Date of publication of application: **09.07.2025**
(21) Application number: 25157210.3
(22) Date of filing: 10.04.2022
(51) Int. Cl.: A61F 2/24

(54) **CATHETER STABILIZATION DEVICES**

(30) Priority: 22.04.2021 US 202163178447 P
(62) Divisional of application: 22723473.9
(71) Applicant: Edwards Lifesciences Innovation (Israel) Ltd., 3079892 Caesarea (IL)
(72) Inventor: Aviv, Ehud, IRVINE, CA, 92614 (US); Chen, Philip Jiun-An, IRVINE, CA, 92614 (US); Shaolian, Eyal, IRVINE, CA, 92614 (US); Heflin, Ernest William, IRVINE, CA, 92614 (US)
(74) Representative: Eisenführ Speiser

(57) **Abstract**

A catheter stabilization system comprises a catheter and a stabilizer. The catheter has a steerable distal end. The stabilizer is coupled with the catheter at a position that is proximal to the steerable distal end. The stabilizer is movable radially outward relative to the catheter to stabilize the catheter during flexing of the distal end of the steerable catheter.

## Description

### CROSS-REFERENCE TO RELATED APPLICATIONS

This application claims the benefit of U.S. Provisional Application No. 63/178,447, filed on April 22, 2021, the contents of which are incorporated by reference in its entirety.

### BACKGROUND

Endovascular delivery systems can be used in various procedures to deliver medical devices or instruments to a target location inside a patient's body that are not readily accessible by surgery or where access without surgery is desirable. The systems described herein can be used to deliver medical devices (e.g., stents, heart valve, grafts, clips, repair devices, valve treatment devices, etc.) to a location in a patient's body.

Access to a target location inside the patient's body can be achieved by inserting and guiding the delivery system through a pathway or lumen in the body, including, but not limited to, a blood vessel, an esophagus, a trachea, any portion of the gastrointestinal tract, a lymphatic vessel, to name a few. Catheters are known in the art and have been commonly used to reach target locations inside a patient's body.

In some procedures, a catheter is used to deliver a device for repairing a native heart valve. The native heart valves (i.e., the aortic, pulmonary, tricuspid, and mitral valves) serve critical functions in assuring the forward flow of an adequate supply of blood through the cardiovascular system. These heart valves can be damaged, and thus rendered less effective, for example, by congenital malformations, inflammatory processes, infectious conditions, disease, etc. Such damage to the valves can result in serious cardiovascular compromise or death. Damaged valves can be surgically repaired or replaced during open heart surgery. However, open heart surgeries are highly invasive, and complications may occur. Transvascular techniques can be used to introduce and implant prosthetic devices in a manner that is much less invasive than open heart surgery. As one example, a transvascular technique useable for accessing the native mitral and aortic valves is the trans-septal technique. The trans-septal technique comprises advancing a catheter into the right atrium (e.g., inserting a catheter into the right femoral vein, up the inferior vena cava and into the right atrium). The septum is then punctured, and the catheter passed into the left atrium. A similar transvascular technique can be used to implant a prosthetic device within the tricuspid valve that begins similarly to the trans-septal technique but stops short of puncturing the septum and instead turns the delivery catheter toward the tricuspid valve in the right atrium. Catheters can be used in procedures involving various implants, such as annuloplasty rings (which can reshape a heart valve), valve repair devices (which can repair existing heart valves), implantable heart valve replacement devices, docking stations for heart valve replacement devices, etc.

### SUMMARY

This summary is meant to provide some examples and is not intended to be limiting of the scope of the invention in any way. For example, any feature included in an example of this summary is not required by the claims, unless the claims explicitly recite the features. Also, the features, components, steps, concepts, etc. described in examples in this summary and elsewhere in this disclosure can be combined in a variety of ways. Various features and steps as described elsewhere in this disclosure can be included in the examples summarized here.

In some implementations, a catheter stabilization system or device includes a catheter and a stabilizer. The catheter has a steerable distal end. The stabilizer is coupled with the catheter at a position that is proximal to the steerable distal end. The stabilizer is movable radially outward relative to the catheter (e.g., from a non-expanded or non-extended position/state/configuration to an actuated or expanded position/state/configuration) to stabilize the catheter during flexing of the distal end of the steerable catheter.

In some implementations, the stabilizer is configured to be actuated to engage vasculature to stabilize the catheter. In some implementations, the stabilizer is configured to be expanded to engage vasculature to stabilize the catheter.

In some implementations, the catheter comprises one or more of a guide sheath, sleeve, steerable catheter, and/or an implant catheter.

In some implementations, the stabilizer is configured such that, in a non-expanded state, the stabilizer is flush with the catheter.

In some implementations, the stabilizer comprises one or more extensions (e.g., one or more legs, one or more skis, one or more fins, one or more inflatable members, a combination of these, etc.) coupled with the catheter. In some implementations, the extensions (e.g., legs, skis, fins, inflatable members, combinations of different types of extensions, etc.) actuate, move, expand, extend, etc. radially outward from the catheter to stabilize the catheter (e.g., from a non-expanded or non-extended position/state/configuration to an actuated or expanded position/state/configuration).

In some implementations, the stabilizer comprises a channel and wire within the channel or multiple wires. The wire or wires can be a shape-set wire or shape-set wires. In some implementations, the wire(s) extends curls back towards the catheter upon being forced from the channel.

In some implementations, the stabilizer comprises a mesh material. In some implementations, the mesh material is a shape-set mesh material. In some implementations, the shape set mesh curls back towards the catheter upon being pushed out of the catheter.

There is also provided in some implementations, a catheter stabilization system or device includes a sleeve, a catheter, and a stabilizer. The catheter has a steerable distal end. The stabilizer is coupled with the sleeve. The stabilizer is movable radially outward relative to the sleeve (e.g., from a non-expanded or non-extended position/state/configuration to an actuated or expanded position/state/configuration) to stabilize the sleeve and/or the catheter during flexing of the distal end of the steerable catheter.

In some implementations, the stabilizer is configured to engage the vasculature.

In some implementations, the catheter comprises at least one of a guide sheath, a steerable catheter, and an implant catheter.

In some implementations, the stabilizer is disposed or positionable proximal to a distal tip of the sleeve.

In some implementations, in a non-expanded state/configuration, the stabilizer is flush with the catheter and/or the sleeve.

In some implementations, the stabilizer comprises one or more extensions coupled with the sleeve and/or with the catheter. In some implementations, the extensions comprise one or more of legs, skis, fins, inflatable members, wires, mesh, etc. In some implementations, the extensions are configured to actuate, move, expand, extend, etc. radially outward (e.g., from a non-expanded or non-extended position/state/configuration to an actuated or expanded position/state/configuration).

In some implementations, the stabilizer comprises a channel and wire within the channel or multiple wires. The wire or wires can be a shape-set wire or shape-set wires. In some implementations, the wire(s) extends curls back towards the catheter upon being forced from the channel.

In some implementations, the stabilizer comprises a mesh material. In some implementations, the mesh material is a shape-set mesh material. In some implementations, the shape set mesh curls back towards the catheter upon being pushed out of the catheter.

In some implementations, a method of stabilizing a catheter comprises advancing one or more of a catheter, a stabilizer, and/or a combination of these through vasculature of a patient. The method includes actuating, expanding, or extending the stabilizer to engage the vasculature. For example, the method can include expanding or extending the stabilizer (e.g., extensions, legs, skis, fins, inflatable portions, one or more other portions of the stabilizer, etc.) radially outward from the catheter to engage the vasculature.

The method can further include flexing or steering a distal end of the catheter while the stabilizer is coupled or engaged with the vasculature, e.g., to perform a treatment (such as a repair or replacement procedure, an annuloplasty procedure, an implantation, etc.).

In some implementations, the method further comprises advancing a sheath through the vasculature of a patient, wherein the sheath is disposed radially outward of the catheter and the stabilizer. In some implementations, the sheath is removed from at least a portion of the vasculature after the catheter and the stabilizer are advanced through the stabilizer.

In some implementations, the catheter and the stabilizer are coupled such that they advance through the vasculature at the same time. In some implementations, the catheter and the stabilizer are uncoupled such that they advance through the vasculature at different times.

In some implementations, actuating, expanding, or extending the stabilizer comprises extending one or more extensions (e.g., legs, skis, fins, inflatable members, combination of different types of extensions, etc.) of the stabilizer radially outward. In some implementations, actuating, expanding, or extending the stabilizer comprises extending one or more legs of the stabilizer radially outward. In some implementations, actuating, expanding, or extending the stabilizer comprises extending one or more skis of the stabilizer radially outward. In some implementations, actuating, expanding, or extending the stabilizer comprises extending one or more fins of the stabilizer radially outward. In some implementations, actuating, expanding, or extending the stabilizer comprises inflating one or more inflatable members of the stabilizer radially outward. In some implementations, actuating, expanding, or extending the stabilizer comprises extending a wire (e.g., a shape-set wire) from a channel of the stabilizer. In some implementations, actuating, expanding, or extending the stabilizer comprises extending a shape-set sheet from a channel of the stabilizer.

The above method(s) can be performed on a living animal or on a simulation, such as on a cadaver, cadaver heart, simulator (e.g., with the body parts, heart, tissue, etc. being simulated), etc.

In some implementations, a catheter stabilization system includes a catheter, a stabilizer, and a valve repair or replacement system, device, or implant. The catheter has a steerable distal end. The stabilizer is coupled with the catheter at a position that is proximal to the steerable distal end. The stabilizer is movable radially outward relative to the catheter to stabilize the catheter during flexing of the distal end of the steerable catheter to position the valve repair or replacement system, device, or implant.

In some implementations, the valve repair or replacement system, device, or implant comprises one or more of a valve repair device, an annuloplasty system, an annuloplasty structure, an annuloplasty ring, an implantable valve replacement device, a docking station for valve a replacement device, etc. Any of the systems, devices, stabilizers herein can be used to deliver the valve repair or replacement system, device, or implant.

In some implementations, the valve repair or replacement system, device, or implant is configured to be delivered transluminally or transvascularly. In some implementations, the valve repair or replacement system, device, or implant includes multiple anchors (e.g., helical anchors, dart-like anchors, staple-like anchors, clips, etc.) that are delivered/anchored or configured to be delivered/anchored at multiple locations of a native heart valve (e.g., at multiple locations around an annulus of a native heart valve of a heart). The stabilizer can be configured to allow precise location and placement of the multiple anchors at the multiple locations. In some implementations, the valve repair or replacement system, device, or implant comprises an annuloplasty system, structure, device, or ring that comprises multiple anchors and a tether or tensioning member that can be tensioned such that a shape and/or size of the annulus of the native heart valve is changed (e.g., by pulling the anchors closer together, etc.). In some implementations, the annuloplasty system, structure, device, or ring comprises spacers that help space the multiple anchors apart and/or help distribute forces between the anchors (e.g., the annuloplasty system, structure, device, or ring can comprise a tether or tensioning member that passes through eyelets of multiple anchors and through a lumen or center of spacers between the anchors.) Precise placement of the anchors is facilitated by the stabilizer by providing more control over the movement of the catheter while delivering the anchors.

A further understanding of the nature and advantages of the present invention are set forth in the following description and claims, particularly when considered in conjunction with the accompanying drawings in which like parts bear like reference numerals.

### BRIEF DESCRIPTION OF THE DRAWINGS

To further clarify various aspects of implementations of the present disclosure, a more particular description of the certain implementations will be made by reference to various aspects of the appended drawings. It is appreciated that these drawings depict only typical implementations of the present disclosure and are therefore not to be considered limiting of the scope of the disclosure. Moreover, while the figures can be drawn to scale for some implementations, the figures are not necessarily drawn to scale for all implementations. Implementations and other features and advantages of the present disclosure will be described and explained with additional specificity and detail through the use of the accompanying drawings in which:
FIG. 1 illustrates a cutaway view of the human heart in a diastolic phase;
FIG. 2 illustrates a cutaway view of the human heart in a systolic phase;
FIG. 3 illustrates an example of a stabilizer in a non-expanded state on a catheter;
FIG. 4 illustrates an example of a stabilizer in an expanded state on a catheter;
FIG. 5 illustrates an example of a stabilizer in a non-expanded state on a catheter that is in a blood vessel;
FIG. 6 illustrates an example of a stabilizer in an expanded state on a catheter that is in a blood vessel;
FIG. 7 illustrates an example of a stabilizer in a non-expanded state on a sleeve that is in a blood vessel;
FIG. 8 illustrates an example of a stabilizer in an expanded state on a sleeve that is in a blood vessel;
FIGS. 9-13 illustrate an example of a catheter stabilization device in a human heart;
FIGS. 14-17 illustrate an example of a stabilizer of a catheter stabilization device;
FIGS. 18-21 illustrate an example of a stabilizer of a catheter stabilization device;
FIGS. 22-24 illustrate an example of a stabilizer of a catheter stabilization device;
FIG. 25 illustrates an example a stabilizer of a catheter stabilization device;
FIG. 26 illustrates an example a stabilizer of a catheter stabilization device;
FIG. 27 illustrates an example a stabilizer of a catheter stabilization device;
FIG. 28 illustrates an example a stabilizer of a catheter stabilization device;
FIG. 29 illustrates an example a stabilizer of a catheter stabilization device;
FIG. 30 illustrates an example a stabilizer of a catheter stabilization device;
FIG. 31 illustrates an example a stabilizer of a catheter stabilization device;
FIGS. 32-34 illustrate deployment of an example a stabilizer of a catheter stabilization device into vasculature;
FIGS. 35-36 illustrate an example a stabilizer of a catheter stabilization system;
FIGS. 37-40 illustrate an example a stabilizer of a catheter stabilization device; and
FIG. 41 illustrates an example a stabilizer of a catheter stabilization device.

### DETAILED DESCRIPTION

The following description refers to the accompanying drawings, which illustrate specific implementations of the present disclosure. The drawings demonstrate several possible configurations of systems, devices, components, and methods that may be used for various aspects and features of the present disclosure. Other implementations having different structures and operation do not depart from the scope of the present disclosure. Specific examples provided herein are not intended to be limiting; for example, steering mechanisms described herein can also be adapted and used to steer other systems and devices not expressly described herein. As one example, various systems, devices, components, and methods are described herein that may relate to steerable catheters. As a further example, Published PCT Patent Application No. WO2020/106705, which is incorporated by reference herein in its entirety, also describes various steerable catheters that can be used with the steering mechanisms, steering elements, and other features described herein.

Example implementations of the present disclosure are directed to devices and methods for steering a flexible delivery system for a medical device, such as a catheter. These example delivery systems provide a wide range of motion for the positioning of a medical device and are versatile, reliable, and easy to use. For example, the delivery systems disclosed herein can be used to position and deploy an implantable medical device for use in the repair of a native heart valve. It should be noted that various implementations of native valve reparation devices and systems for delivery are disclosed herein, and any combination of these options can be made unless specifically excluded. In other words, individual components of the disclosed devices and systems can be combined unless mutually exclusive or otherwise physically impossible.

As described herein, when one or more components are described as being connected, joined, affixed, coupled, attached, or otherwise interconnected, such interconnection may be direct as between the components or may be indirect such as through the use of one or more intermediary components. Also as described herein, reference to a "member," "component," or "portion" shall not be limited to a single structural member, component, or element but can include an assembly of components, members, or elements. Also as described herein, the terms "substantially" and "about" are defined as at least close to (and includes) a given value or state (preferably within 10% of, more preferably within 1% of, and most preferably within 0.1% of).

When introducing a catheter into the heart, it can be necessary to locate and use existing anatomy to support, secure, or otherwise stabilize the catheter. When navigating around the mitral valve, for example, the catheters can be stabilized by the septum for the subsequent procedure. In many cases, however, the anatomy is not accessible to support, secure, or otherwise stabilize the catheter. For example, in the inferior vena cava, heart anatomy may be too large to stabilize the catheter when attempting to treat the tricuspid valve. In cases like this, navigating around the heart can be challenging due to a lack of support for the delivery system. The present application discloses example implementations of stabilizing arrangements that stabilize components of a delivery system, such as a delivery sheath, a steerable catheter, a device catheter, a device pusher, a guidewire, etc. during delivery of a device, such as a valve repair device, a valve repair device, a heart wall remodeling system, etc.

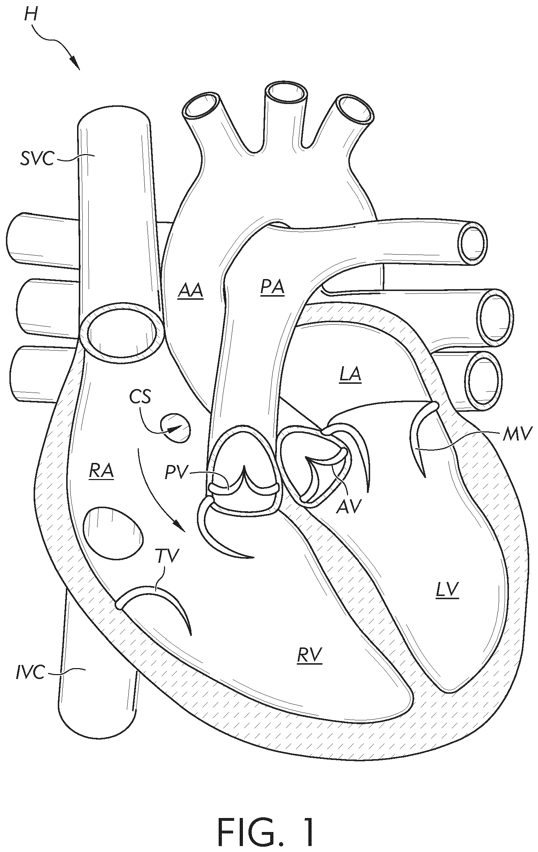

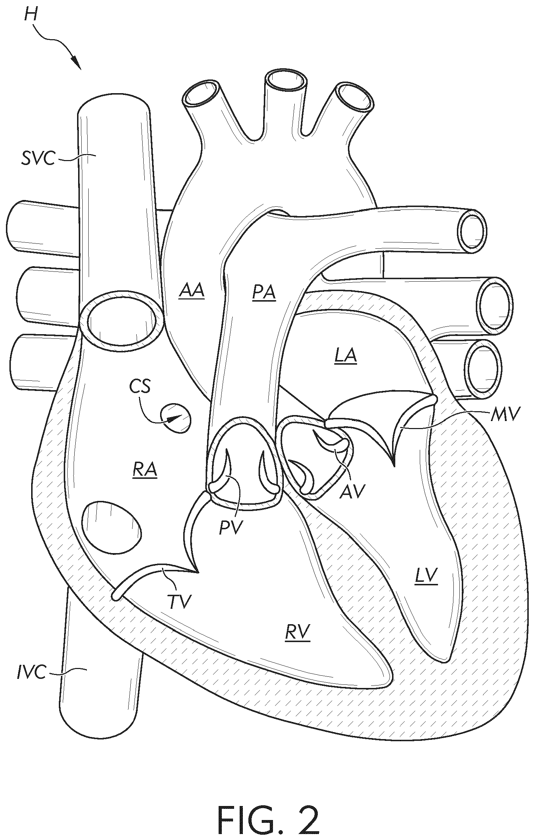

Figures 1 and 2 are cutaway views of the human heart H in diastolic and systolic phases, respectively. The right ventricle RV and left ventricle LV are separated from the right atrium RA and left atrium LA, respectively, by the tricuspid valve TV and mitral valve MV, respectively; i.e., the atrioventricular valves. The aortic valve AV separates the left ventricle LV from the ascending aorta AA, and the pulmonary valve PV separates the right ventricle from the pulmonary artery PA. Each of these valves has flexible leaflets (not shown) extending inward across the respective orifices that come together or "coapt" in the flow stream to form the one-way, fluid-occluding surfaces. The native valve repair systems of the present application are described primarily with respect to the tricuspid valve TV. Therefore, anatomical structures of the right atrium RA and right ventricle RV will be explained in greater detail. It should be understood that the devices described herein may also be used in repairing other native valves, e.g., the devices can be used in repairing the mitral valve MV, the aortic valve AV, and the pulmonary valve PV.

Deoxygenated blood enters the right atrium RA through three major veins: the superior vena cava SVC, the inferior vena cava IVC and the coronary sinus CS. The superior vena cava SVC returns all of the blood to the heart from tissues superior to the heart in the head, neck, arms, and upper thorax. Likewise, the inferior vena cava IVC returns blood to the heart from tissues inferior to the heart, including the legs, abdomen, and lower thorax. Blood from the exterior of the heart itself is collected in the coronary sinus CS to be returned to the interior of the heart. During the diastolic phase, or diastole, seen in Figure 1, blood returning from the veins flows into the right atrium RA and through the tricuspid valve TV to the right ventricle RV. The heart next goes into systole, or contraction, seen in Figure 2, to pump blood from the right ventricle RV to the lungs. The closing of the tricuspid valve TV blocks blood attempting to flow back to the right atrium from the right ventricle RV, so that blood is efficiently pumped in one direction only.

The left atrium LA receives oxygenated blood from the lungs. During the diastolic phase, blood in the left atrium LA moves through the mitral valve MV and into the left ventricle LV by expansion of the left ventricle LV. In the systolic phase, or systole, seen in Figure 2, the left ventricle LV contracts to force the blood through the aortic valve AV and ascending aorta AA into the body. During systole, the leaflets of the mitral valve MV close to prevent the blood from regurgitating from the left ventricle LV back into the left atrium LA.

In some implementations, the devices described by the present application are used during the repair or replacement of a native heart valve, such as the Mitral Valve, the Tricuspid Valve, the Pulmonary Valve, or the Aortic Valve. In the present application, the stabilizers are shown being used to navigate to the tricuspid valve TV. However, the stabilizers can be used with any system that accesses any of the native valves of the heart. In some examples, the stabilizers are configured to work with utilize existing heart anatomy to provide support to the delivery system, such as one or more catheters of the delivery system. In the illustrated examples, the catheter and related devices can help repair and/or replace the leaflets of the tricuspid valve TV to prevent blood from regurgitating from the right ventricle RV and back into the right atrium RA.

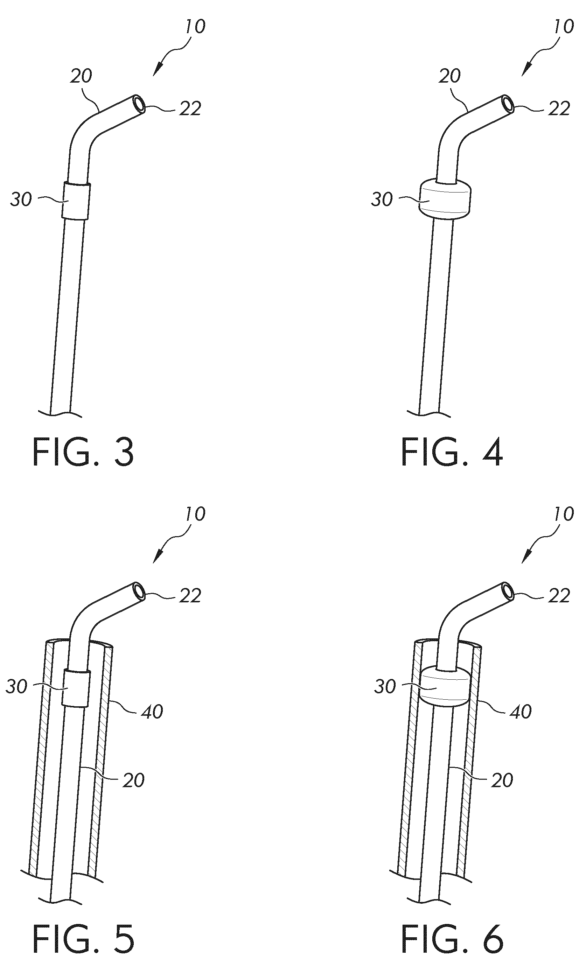

Referring to Figures 3 and 4, an example support structure is shown that is configured to secure, support, or otherwise stabilize the catheter against existing patient vasculature. In the example illustrated by Figures 3 and 4, a catheter stabilizing system 10 can include a catheter 20 comprising a stabilizer 30 or can include a stabilizer 30 configured to be used with a catheter 20. The stabilizer 30 can be used on a wide variety of catheters. In some implementations, the catheter 20 can be a guide sheath, steerable catheter, an implant catheter, and implant pusher, etc.

The catheter stabilizing device 10 can include a stabilizer 30 having a wide variety of forms, some of which are described herein. The stabilizer 30 can include any structure that can be actuated, expanded, extended, secured, anchored, leveraged, or can otherwise be used to stabilize the catheter 20 during the flexing of the catheter 20. The stabilizer 30 can be actuated (e.g., expanded, extended, secured, anchored, leveraged, stabilized, etc.) in a variety of ways against patient vasculature to stabilize the catheter 20 during various procedures. In some implementations, the stabilizer 30 is configured to expand, extend, etc. to various diameters and/or increased size as needed based on the size of the patient's vasculature. The stabilizer 30 can be configured to thereafter contract to a diameter and/or reduced size that is smaller than the maximum diameter and/or size, as needed based on the location of the catheter 20 in the patient and the vasculature that is being used for stabilization.

In some implementations, in a compressed or reduced size state/configuration, the stabilizer 30 can be flush or substantially flush with the catheter 20. In some instances, in the non-expanded, compressed, or reduced size state/configuration, the stabilizer 30 can extend partially radially outward from the catheter. When the stabilizer 30 is actuated, expanded, extended, etc. to an actuated state/configuration or expanded state/configuration, the stabilizer 30 can extend from the catheter 20 to the vasculature of the patient.

With reference to Figures 3-6, the stabilizer 30 can be coupleable to the catheter 20 or can be integral with the catheter 20 itself. In some implementations, the stabilizer 30 can be located proximal to the distal tip 22 of the catheter 20. The stabilizer 30 can be located in various other locations of the catheter 20 as needed based on the location of the catheter 20 in the patient and the vasculature that is being used for stabilization. The stabilizer 30 can comprise a non-expanded, compressed, or reduced size state/configuration (see Figure 3) as well as an actuated or expanded state/configuration (Figure 4).

The stabilizer 30 can take a wide variety of different forms. For example, in some implementations, the stabilizer 30 can be an inflatable device, such as a bladder or a balloon, a mechanical linkage, one or more extendable members, etc. In some implementations, the stabilizer can be any structure or can comprise any structure that can move from a retracted or compressed condition to an actuated or expanded condition.

With reference to Figures 5-6, the catheter 20 is located in a patient body, for example, in a blood vessel 40. In some implementations, the catheter 20 can be positioned through the blood vessel or vasculature 40. For example, the blood vessel or vasculature 40 can be any vessel of the circulatory system, heart, any cavity of the heart, any vessel or vasculature that is in direct communication with the heart, etc. At the desirable location, the stabilizer 30 can be actuated (e.g., expanded, extended, etc.) (Figure 6) such that it couples with or engages the blood vessel or vasculature 40 to stabilize the catheter 20 within the blood vessel or vasculature 40. Once the stabilizer 30 couples with or engages the blood vessel or vasculature 40, the tip 22 of the catheter can be accurately positioned and stabilized to deliver or deploy the repair or replacement device.

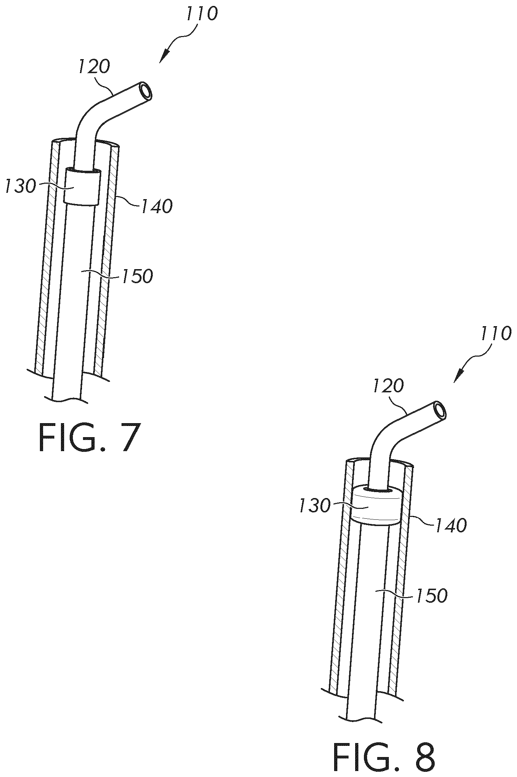

Figures 7 and 8 illustrate an example of a delivery system that includes a stabilizer or catheter stabilizing device 110. With reference to Figures 7-8, the catheter stabilizing device 110 can include a stabilizer 130 located or coupleable on a sleeve 150. Figure 7 illustrates the sleeve 150 comprising the stabilizer 130 in a non-expanded state/configuration or reduced size state/configuration, and Figure 8 illustrates the sleeve 150 comprising the stabilizer 130 in an actuated or expanded state/configuration. The catheter 120 is in the sleeve in this example. With reference to Figure 8, the stabilizer 130 can be actuated or expanded such that it couples with the blood vessel 140 to stabilize the sleeve 150, and thus, the catheter 120 within the blood vessel 140.

In any of the implementations herein, the catheter stabilizing device can include a stabilizer located on the sleeve as well as on the catheter. This could be beneficial if there are multiple structures of different sizes which can be used to stabilize the catheter. Any of the implementations described herein can comprise devices wherein the stabilizer is located on the catheter 20 (see Figures 3-6) or on the sleeve (see Figures 7-8), or on both the catheter and the sleeve.

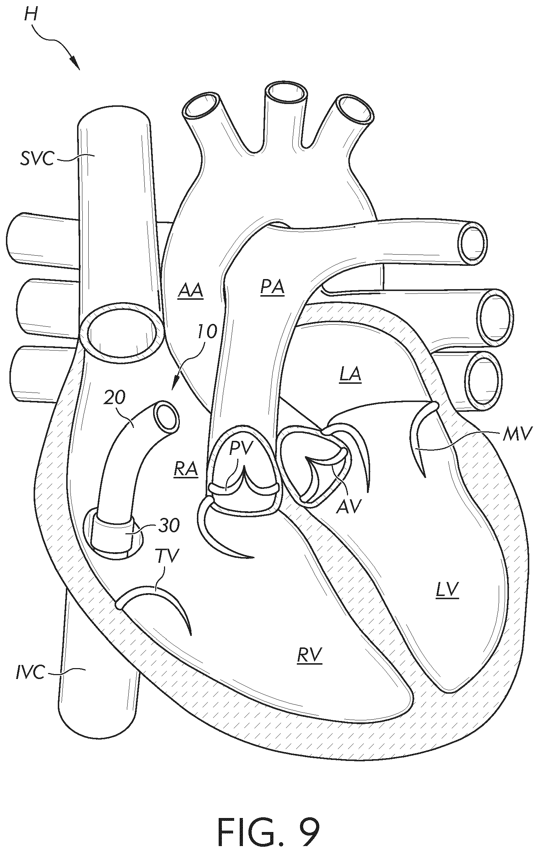

The catheter stabilizing device 10 can go into any vasculature to provide stability to access any area of the heart, including, but not limited to the mitral valve MV, the aortic valve AV, the pulmonary valve PV, and the tricuspid valve TV. In some implementations, with reference to Figures 9-13, a catheter stabilizing device 10 is deployed through the inferior vena cava IVC to access the tricuspid valve TV. This can help stabilize the catheter within the IVC to allow more precise treatment at the tricuspid valve (e.g., without the catheter moving and thereby moving the location of the distal end of the catheter from a desired treatment location). With reference to Figure 9, the catheter 20 is positioned within the right atrium RA while the stabilizer 30 is positioned within the inferior vena cava IVC.

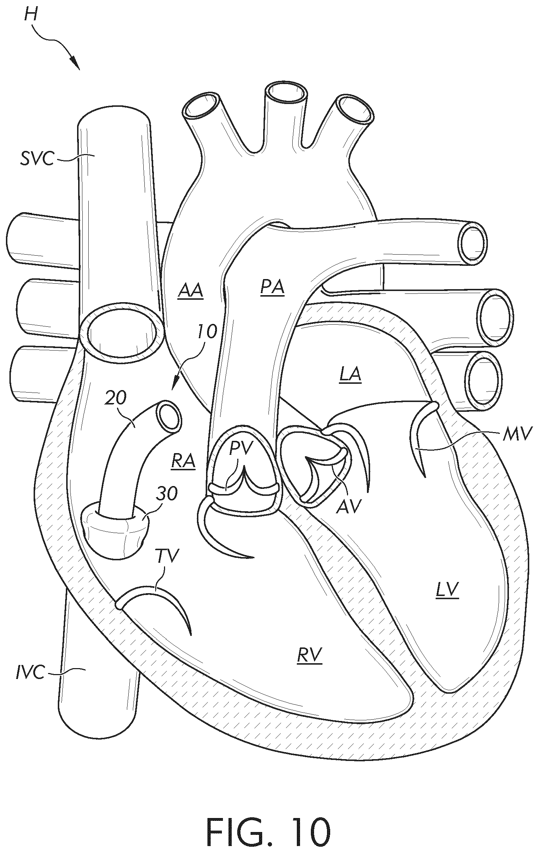

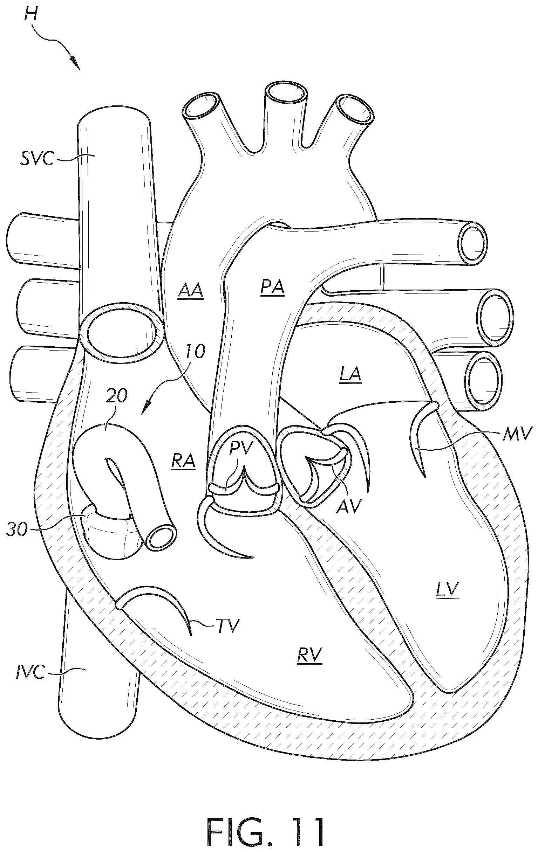

With reference to Figure 10, the stabilizer 30 can be actuated or expanded to an actuated or expended state/configuration wherein the stabilizer 30 (e.g., an outer surface, extension(s), leg(s), ski(s), fin(s), other portion, etc.) is pressed against an inner surface or region of the inferior vena cava IVC to stabilize the catheter 20 within the inferior vena cava IVC. With reference to Figure 11, the catheter 20 can then be repositioned or steered to get to a desired position. The steering of the catheter 20 is supported by the stabilization of the stabilizer 30 against the inferior vena cava IVC.

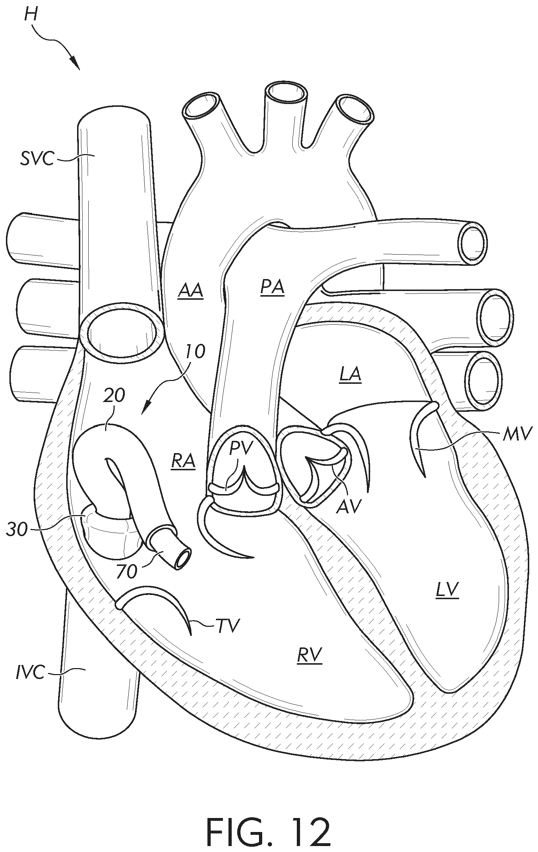

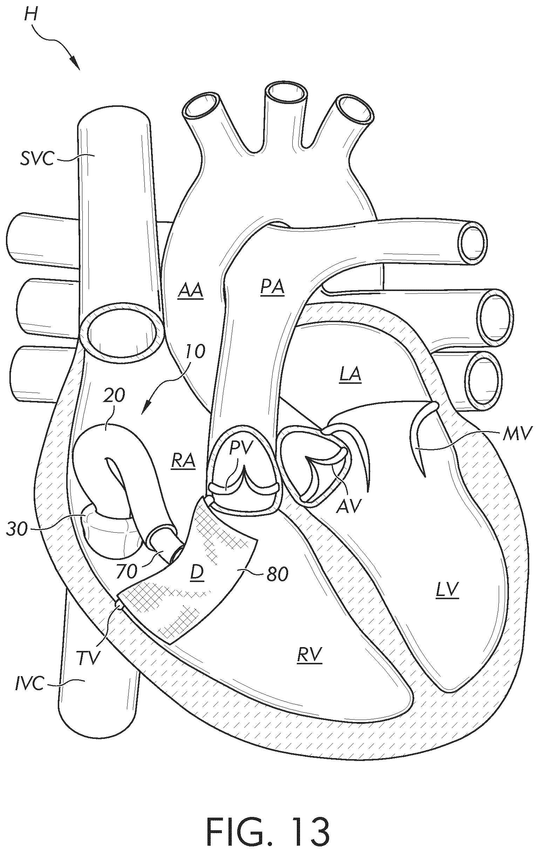

With reference to Figure 12, a delivery catheter 70 is introduced through the catheter 20. With reference to Figure 13, a system, device, or implant 80 can be introduced through the delivery catheter 70 (and or with any of the systems, devices, catheters, etc. herein) to assist in the repair or replacement of the tricuspid valve or other valves.

The system, device, or implant 80 can comprise any device for repairing or replacing any portion of any heart valve. Examples of systems, devices, or implants 80 include, but are not limited to, annuloplasty structures, annuloplasty systems, annuloplasty rings, valve repair devices, valve replacement devices, and docking stations for heart valve replacement devices. As some examples, system, device, or implant 80 can comprise any of the systems, devices, and/or implants in US Patent 9,636,224, PCT Application No. PCT/IB2020/060044, US Patent Application No. 63/130,364, US Patent Application No. 63/113,430, and US Patent Application No. 63/124,704, each of the foregoing applications is incorporated by reference herein in their entireties for all purposes.

In some implementations, the system, device, or implant 80 is configured to be delivered transluminally or transvascularly. In some implementations, the system, device, or implant 80 includes multiple anchors (e.g., helical anchors, dart-like anchors, staple-like anchors, clips, etc.) that are delivered/anchored or configured to be delivered/anchored at multiple locations of a native heart valve (e.g., at multiple locations around an annulus of a native heart valve of a heart). The stabilizer can be configured to allow precise location and placement of the multiple anchors at the multiple locations. In some implementations, the system, device, or implant 80 comprises an annuloplasty system, structure, device, or ring that comprises multiple anchors and a tether or tensioning member that can be tensioned such that a shape and/or size of the annulus of the native heart valve is changed (e.g., by pulling the anchors closer together, etc.). In some implementations, the annuloplasty system, structure, device, or ring comprises spacers that help space the multiple anchors apart and/or help distribute forces between the anchors (e.g., the annuloplasty system, structure, device, or ring can comprise a tether or tensioning member that passes through eyelets of multiple anchors and through a lumen or center of spacers between the anchors.) Precise placement of the anchors is facilitated by the stabilizer by providing more control over the movement of the catheter while delivering the anchors.

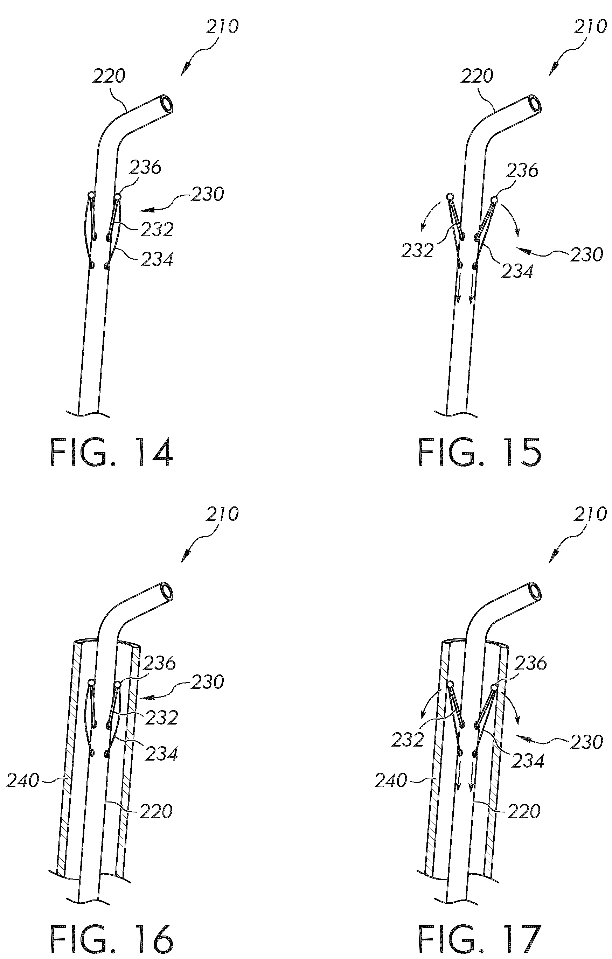

As is mentioned above, the stabilizers can take a wide variety of different forms. Figures 14-17 illustrate an example of a catheter stabilizing device. In the example illustrated by Figures 14-17, a catheter stabilizing device 210 can comprise a stabilizer 230. The stabilizer 230 can be located on the catheter and/or on the sleeve, as described herein. The stabilizer 230 can include one or more extensions or legs 232. Any structure that can move the legs 232 from a more radially inward or non-extended position/configuration to a more radially outward or extended position/configuration can be used. In some implementations, the legs 232 can be attached to wires 234 on the distal end 236 of the legs 232. With reference to Figures 15 and 17, an actuator (not shown) can push or pull on the wires 234, which in turn pull the distal end 236 of the legs 232 radially outward relative to the catheter 220 to an actuated or expanded/extended state/configuration. With reference to Figure 17, when the legs 232 are pulled outward, they reach and press against the blood vessel or vasculature 240 to stabilize the catheter 220 within the blood vessel or vasculature 240. As shown in Figures 14-17, the stabilizer 230 can include two legs 232, however any number of legs 232 can be used.

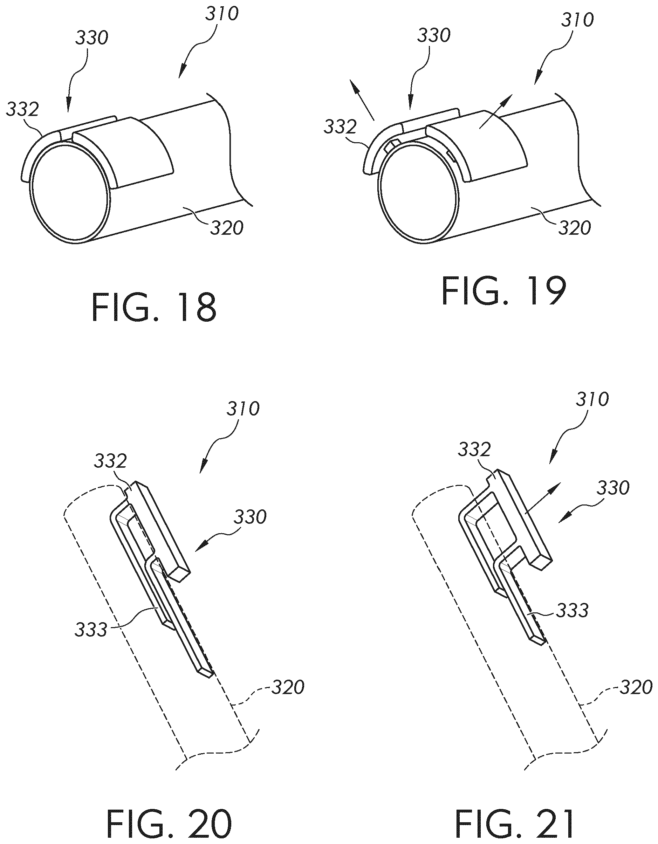

Figures 18-21 illustrate an example of a catheter stabilizing device. In the example illustrated by Figures 18-21, the catheter stabilizing device 310 can comprise a stabilizer 330. The stabilizer 330 can be located on the catheter and/or on the sleeve, as described herein. The stabilizer 330 can include one or more extensions or skis 332. The skis 332 can be moved radially outward relative to the catheter 320 to an actuated or expanded state/configuration. Any structure that can move the skis 332 from a more radially inward or non-extended position/configuration to a more radially outward or extended position/configuration can be used. In some implementations, the skis 332 can be attached to wires 333 (see Figures 19 and 20). With reference to Figure 19 and 21, an optional actuator (not shown) can push on the wires 333, which in turn push the skis 332 radially outward relative to the catheter 320 to an actuated or expanded/extended state/configuration. When the skis 332 are pushed outward, they can reach and press against a blood vessel or other vasculature structure to stabilize the catheter 320 within that structure. As shown in Figures 18-21, the stabilizer 330 can include two skis 332, however any number of skis 332 can be used.

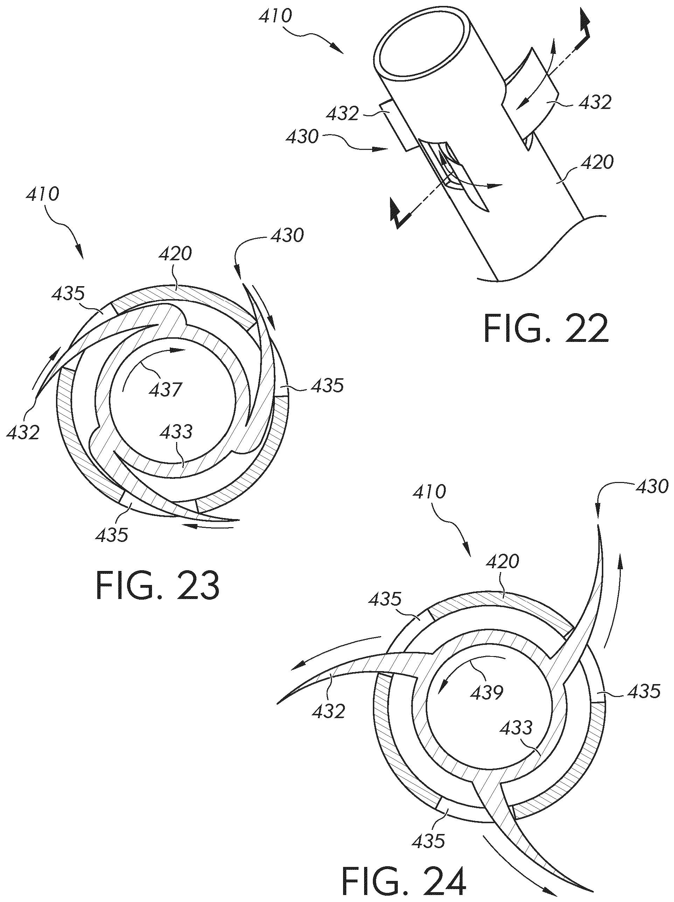

Figures 22-24 illustrate an example of a catheter stabilizing device. In the example illustrated by Figures 22-24, a catheter stabilizing device 410 can comprise a stabilizer 430. The stabilizer 430 can extend from the catheter and/or extend from the sleeve. The stabilizer 430 can include one or more extensions or fins 432. The fins 432 can be moved radially outward relative to the catheter 420 to an actuated or expanded state/configuration. Any structure that can allow the fins 432 to move from a more radially inward or non-extended position/configuration to a more radially outward or extended position/configuration can be used. In the illustrated example, the fins 432 are attached to a central shaft 433 and extend through openings 435 of the outer wall of the catheter or sleeve. Referring to Figure 23, when the shaft 433 is rotated in the direction 437, the fins 432 are drawn through the openings 435 into the catheter 420 by the shaft 433 to retract the fins. Referring to Figure 24, when the shaft 433 is rotated in the direction 439, the fins 432 are pushed out of the openings 435 of the catheter 420 by the shaft 433 to extend the fins to an actuated or extended state/configuration. In some implementations, an actuator can be used to extend and retract the fins 432. When the fins 432 are pushed outward, they can reach and press against a blood vessel or other vasculature structure to stabilize the catheter 420 within that structure. As shown in Figures 22-24, the stabilizer 430 can include three fins 432, however two, four, five, or any number of fins 432 can be used.

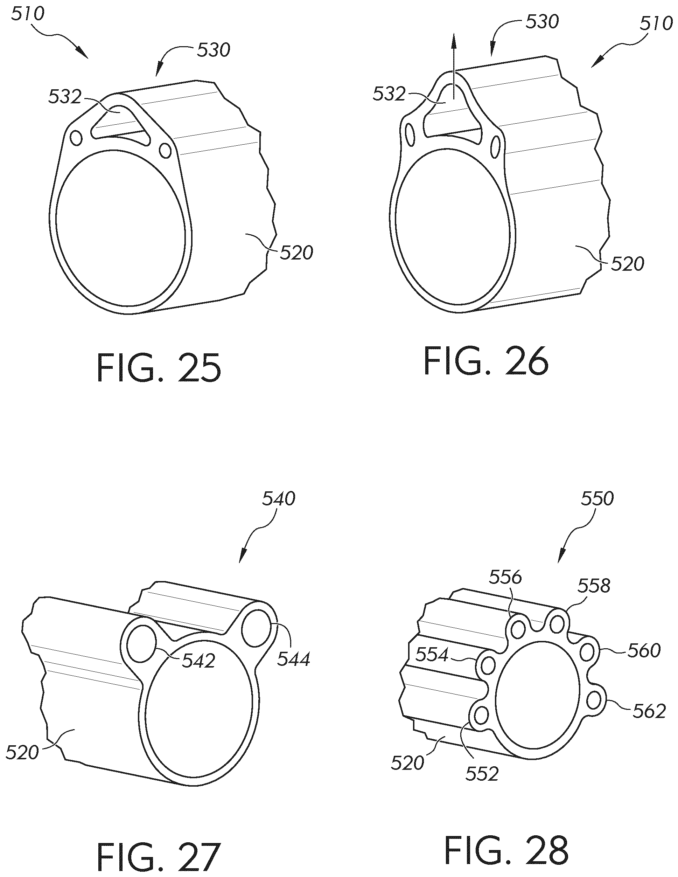

Figures 25-28 illustrate examples of catheter stabilizing devices. In the example illustrated by Figures 25-28, the catheter stabilizing device 510 can comprise a stabilizer 530. The stabilizer 530 can be located on the catheter or on the sleeve, as described herein. The stabilizer 530 can include one or more inflatable members or portions 532. The inflatable member 532 can be inflated to expand radially outward relative to the catheter 520 to an actuated or expanded state/configuration. Any structure that can allow the inflatable member 532 to inflate or otherwise expand from a more radially inward or non-extended position/configuration to a more radially outward or extended position/configuration can be used. In some implementations, with reference to Figure 26, a pump, syringe, etc. can force fluid, such as gas (e.g., air, nitrogen, etc.) or liquid (e.g., water, saline solution, etc.) into the inflatable member 532 to inflate the inflatable member 532. As a result, the inflatable member 532 expands radially outward relative to the catheter 520. When the inflatable member(s) 532 are inflated and expand outward, they can reach and press against a blood vessel or other vasculature structure to stabilize the catheter 520 within that structure. As shown in Figures 25-26, the stabilizer 530 can include one inflatable member 532. With reference to Figure 27, the stabilizer 540 can include two inflatable members 542, 544. With reference to Figure 28, the stabilizer 550 can include six inflatable members 552, 554, 556, 558, 560, and 562. In some implementations, other numbers of inflatable member can be used.

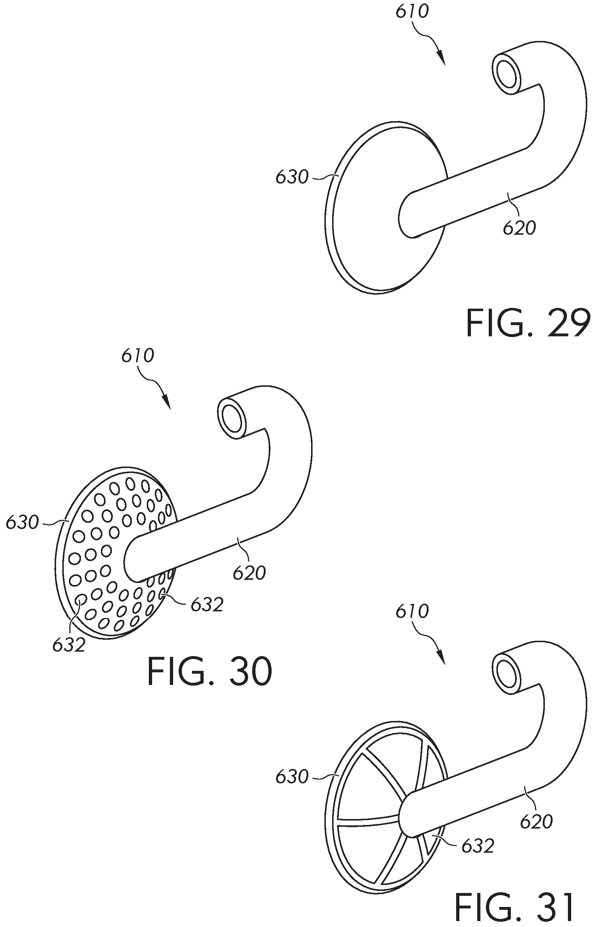

Figures 29-31 illustrate an example of a catheter stabilizing device. In the example illustrated by Figures 29-31, the catheter stabilizing device 610 can comprise a flexible stabilizer 630 that is disposed radially about the catheter 620. The stabilizer 630 can be located on the catheter and/or on the sleeve. The stabilizer 630 can be made of a flexible material, for example silicon, foam, polymer, shape memory material, etc. The stabilizer 630 can comprise perforations 632 that are circular (see Figure 30) or triangular (see Figure 31) or another shape, which allow fluid to enter and/or exit the patient's vasculature. The perforations of the stabilizer can be various other shapes and sizes as needed based on the location of the catheter 620 in the patient and the vasculature that is being used for stabilization.

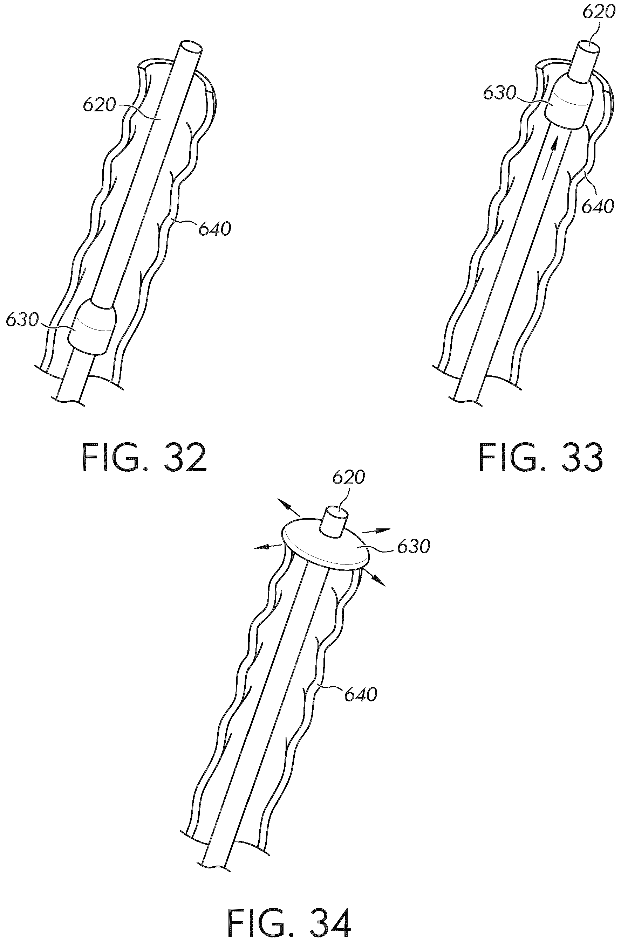

With reference to Figures 32-34, the catheter stabilizing device 610 can be advanced through the vasculature 640 of the patient. Specifically, the catheter 620 can be first introduced through the vasculature 640 of the patient. Thereafter, the stabilizer 630 can be moved distally over the catheter 620 until it reaches the opening of the vasculature 640, such as the opening of the inferior vena cava IVC, or other proper position to actuate or expand. The stabilizer 630 then actuates or expands, extends, etc. and engages the vasculature 640 of the patient to stabilize the catheter 620 for further use.

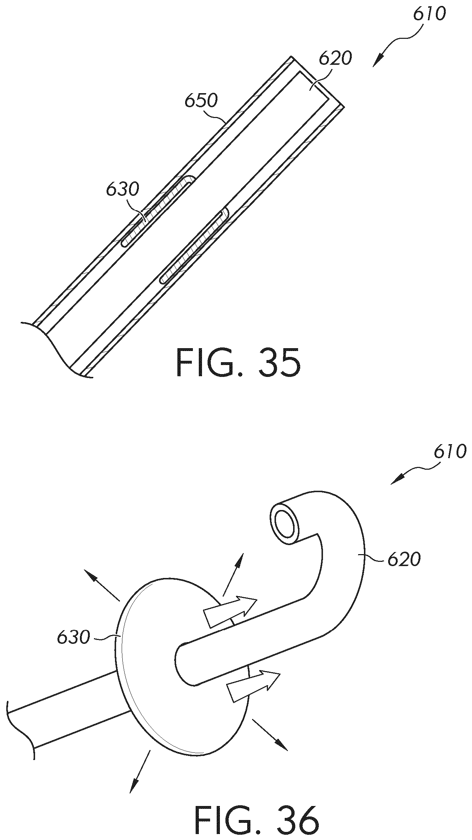

In some implementations, with reference to Figure 35, the catheter stabilizing device 610 can comprise a sheath 650. The catheter 620 and stabilizer 630 can be placed within the sheath 650, and the catheter 620, stabilizer 630, and sheath 650 can be advanced through the vasculature of the patient. With reference to Figure 36, once the catheter stabilizing device is in the proper position, such as at the opening of the vasculature, the sheath can be removed, thereby allowing the stabilizer 630 to engage the vasculature and stabilize the catheter for further use.

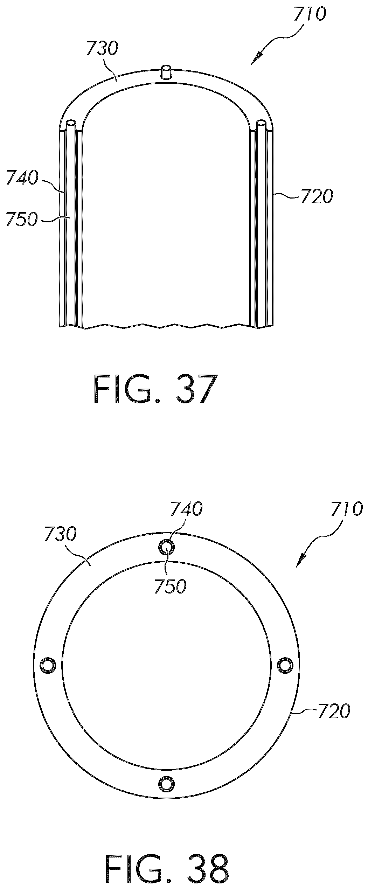

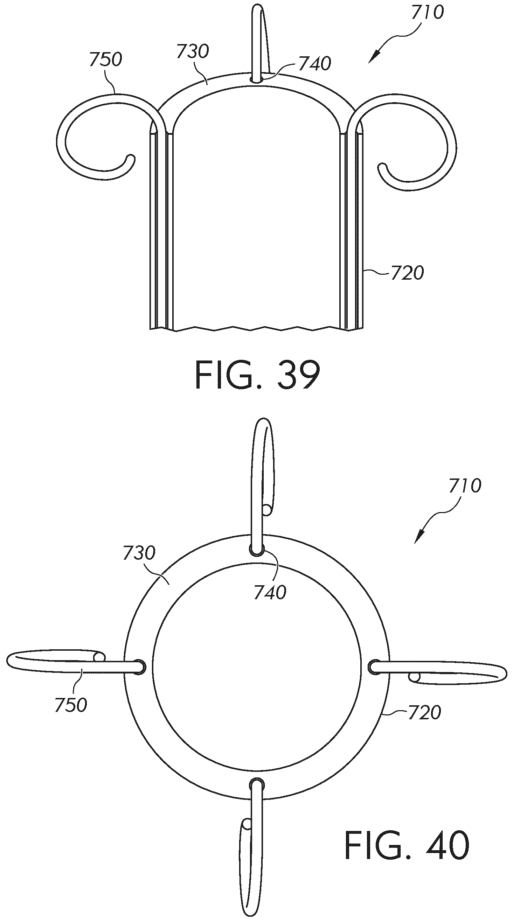

Figures 37-40 illustrate an example of a catheter stabilizing device. In the example illustrated by Figures 37-40, the catheter stabilizing device 710 can comprise a stabilizer 730 that includes channels 740 that comprise wires 750. The wires 750 can be shape-set so that when the wires advance through the distal end of the stabilizer 730, the wires twist or turn according to the predetermined shape. The wires 750, when engaged, can form a variety of shapes, based on the location of the catheter 720 in the patient and the vasculature that is being used for stabilization. The wires 750 can, for example, extend and curl back towards the catheter 720. The wires 750 can be pushed out of the catheter to reach and press against a blood vessel or other vasculature structure to stabilize the catheter 720 within that structure and thereby stabilize the catheter. The channels 740 can comprise a variety of shapes and sizes. For example, with reference to Figures 37-40, the channels 740 can have a substantially circular cross section. The stabilizer 730 can include any number of different channels. For example, with reference to Figures 37-41, the stabilizer 730 can include four channels 740. The wires can be configured in a variety of ways, e.g., solid, composite, braided, woven, mesh, etc.

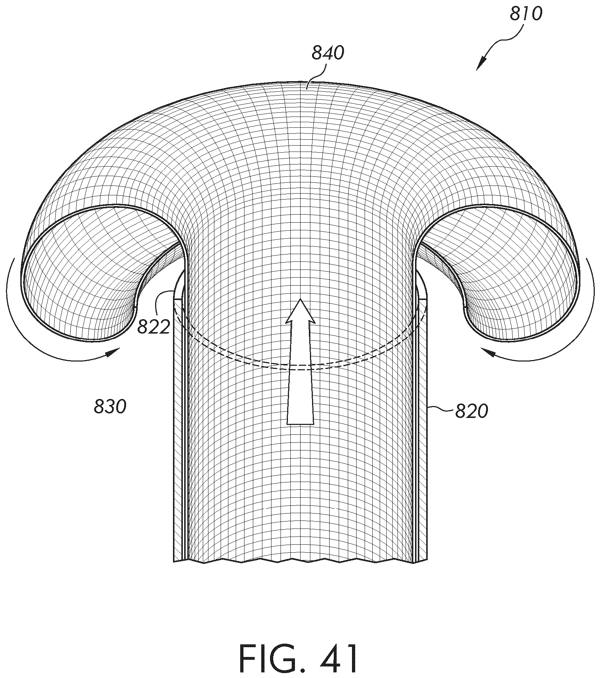

Figure 41 illustrates an example of a catheter stabilizing device. In the example illustrated by Figure 41, the catheter stabilizing device 810 can comprise a stabilizer 830 that includes a shape set mesh material 840. The shape set mesh material 840 can be shape-set so that when it advances through the distal end 822 of the catheter 820, the mesh 840 twists, turns, or curls according to the predetermined shape. The shape set mesh material 840 can comprise a woven or braised wire material. The shape set mesh material 840, when advanced out of the catheter or tube can take a variety of shapes, based on the location of the catheter in the patient and the vasculature that is being used for stabilization. The shape set mesh material 840 can, for example, extend and curl back towards the catheter 820. The shape set mesh material 840 can reach and press against a blood vessel or other vasculature structure to stabilize the catheter within that structure. The mesh can be braided, woven, configured in other ways, etc.

While various inventive aspects, concepts and features of the disclosures may be described and illustrated herein as embodied in combination in the examples, these various aspects, concepts, and features may be used in many alternative embodiments, either individually or in various combinations and sub-combinations thereof. Unless expressly excluded herein all such combinations and sub-combinations are intended to be within the scope of the present application. Still further, while various alternative embodiments as to the various aspects, concepts, and features of the disclosures-such as alternative materials, structures, configurations, methods, devices, and components, alternatives as to form, fit, and function, and so on-may be described herein, such descriptions are not intended to be a complete or exhaustive list of available alternative embodiments, whether presently known or later developed. Those skilled in the art may readily adopt one or more of the inventive aspects, concepts, or features into additional embodiments and uses within the scope of the present application even if such embodiments are not expressly disclosed herein.

Additionally, even though some features, concepts, or aspects of the disclosures may be described herein as being a preferred arrangement or method, such description is not intended to suggest that such feature is required or necessary unless expressly so stated. Still further, example or representative values and ranges may be included to assist in understanding the present application, however, such values and ranges are not to be construed in a limiting sense and are intended to be critical values or ranges only if so expressly stated.

Moreover, while various aspects, features and concepts may be expressly identified herein as being inventive or forming part of a disclosure, such identification is not intended to be exclusive, but rather there may be inventive aspects, concepts, and features that are fully described herein without being expressly identified as such or as part of a specific disclosure, the disclosures instead being set forth in the appended claims. Descriptions of example methods or processes are not limited to inclusion of all steps as being required in all cases, nor is the order that the steps are presented to be construed as required or necessary unless expressly so stated. The treatment techniques, methods, operations, steps, etc. described or suggested herein can be performed on a living animal or on a non-living simulation, such as on a cadaver, cadaver heart, simulator (e.g., with the body parts, tissue, etc. being simulated), etc. The words used in the claims have their full ordinary meanings and are not limited in any way by the description of the embodiments in the specification.

The invention further comprises the following embodiments:
1. A catheter stabilization system comprising:
   a catheter having a steerable distal end;
   a stabilizer coupled or coupleable to the catheter at a position that is proximal to the steerable distal end; and
   wherein the stabilizer is movable radially outward relative to the catheter to stabilize the catheter during flexing of the steerable distal end of the catheter.
2. The catheter stabilization system of embodiment 1, wherein the stabilizer expands and engages vasculature to stabilize the catheter.
3. The catheter stabilization system of any one of embodiments 1-2, wherein the catheter comprises one or more of a guide sheath, steerable catheter, and an implant catheter.
4. The catheter stabilization system of any one of embodiments 1-3, wherein in a non-expanded state, the stabilizer is flush with the catheter.
5. The catheter stabilization system of any one of embodiments 1-4, wherein the stabilizer comprises one or more legs coupled with the catheter.
6. The catheter stabilization system of embodiment 5, wherein the one or more legs move radially outward from the catheter to stabilize the catheter.
7. The catheter stabilization system of any one of embodiments 1-6, wherein the stabilizer comprises one or more skis coupled with the catheter.
8. The catheter stabilization system of embodiment 7, wherein the one or more skis can move radially outward.
9. The catheter stabilization system of any one of embodiments 1-8, wherein the stabilizer comprises one or more fins coupled with the catheter.
10. The catheter stabilization system of embodiment 9, wherein the one or more fins move radially outward to stabilize the catheter.
11. The catheter stabilization system of any one of embodiments 1-10, wherein the stabilizer comprises one or more inflatable members coupled with the catheter.
12. The catheter stabilization system of embodiment 11, wherein the one or more inflatable members expand radially outward upon inflation to stabilize the catheter.
13. The catheter stabilization system of any one of embodiments 1-12, wherein the stabilizer comprises a channel and a shape-set wire within the channel.
14. The catheter stabilization system of embodiment 13, wherein the shape-set wire curls back towards the catheter upon being forced from the channel.
15. The catheter stabilization system of any one of embodiments 1-14, wherein the stabilizer comprises a shape-set mesh material.
16. The catheter stabilization system of embodiment 15, wherein the shape-set mesh material curls back towards the catheter upon being pushed out of the catheter.
17. The catheter stabilization system of any one of embodiments 1-16, further comprising a valve repair or replacement device configured to be delivered to a native heart valve by the catheter.
18. The catheter stabilization system of embodiment 17, wherein the valve repair or replacement device comprises at least one of a valve repair device, a valve replacement device, an annuloplasty system, an annuloplasty structure, an annuloplasty ring, and a docking station.
19. A catheter stabilization system comprising:
   a catheter having a steerable distal end;
   a sleeve disposed radially around the catheter;
   a stabilizer coupled with the sleeve; and
   wherein the stabilizer is movable radially outward relative to the sleeve to stabilize the sleeve and the catheter during flexing of the distal end of the steerable catheter.
20. The catheter stabilization system of embodiment 19, wherein the stabilizer is configured to engage the vasculature.
21. The catheter stabilization system of any one of embodiments 19-20, wherein the catheter comprises at least one of a guide sheath, a steerable catheter, and an implant catheter.
22. The catheter stabilization system of any one of embodiments 19-21, wherein the stabilizer is disposed proximal to a distal tip of the sleeve.
23. The catheter stabilization system of any one of embodiments 19-22, wherein in a non-expanded state, the stabilizer is flush with the catheter.
24. The catheter stabilization system of any one of embodiments 19-23, wherein the stabilizer comprises one or more legs coupled with the catheter.
25. The catheter stabilization system of embodiment 24, wherein the legs are configured to move radially outward.
26. The catheter stabilization system of any one of embodiments 19-25, wherein the stabilizer comprises one or more skis coupled with the catheter.
27. The catheter stabilization system of embodiment 26, wherein the skis are configured to move radially outward.
28. The catheter stabilization system of any one of embodiments 19-27, wherein the stabilizer comprises one or more fins coupled with the catheter.
29. The catheter stabilization system of embodiment 28, wherein the fins are configured to move radially outward.
30. The catheter stabilization system of any one of embodiments 19-29, wherein the stabilizer comprises one or more inflatable members coupled with the catheter.
31. The catheter stabilization system of embodiment 30, wherein the inflatable members are configured to expand radially outward upon inflation.
32. The catheter stabilization system of any one of embodiments 19-31, wherein the stabilizer comprises a channel and a shape-set wire within the channel.
33. The catheter stabilization system of embodiment 32, wherein the wire curls back towards the catheter when the wire is extended from the catheter.
34. The catheter stabilization system of any one of embodiments 19-33, wherein the stabilizer comprises a shape-set mesh material.
35. The catheter stabilization system of embodiment 34, wherein the shape set mesh curls back towards the catheter upon being pushed out of the catheter.
36. The catheter stabilization system of any one of embodiments 19-35, further comprising a valve repair or replacement device or implant configured to be delivered to a native heart valve by the catheter.
37. The catheter stabilization system of embodiment 36, wherein the valve repair or replacement device comprises at least one of a valve repair device, a valve replacement device, an annuloplasty system, an annuloplasty structure, an annuloplasty ring, and a docking station.
38. A method of stabilizing a catheter, comprising:
   advancing a catheter through vasculature of a patient;
   advancing a stabilizer through the vasculature of a patient, wherein the stabilizer is coupled with the catheter;
   expanding the stabilizer radially outward from the catheter to engage the vasculature; and
   flexing a distal end of the catheter while the stabilizer is coupled with the vasculature.
39. The method of embodiment 38, further comprising advancing a sheath through the vasculature of a patient, wherein the sheath is disposed radially outward of the catheter and the stabilizer.
40. The method of any one of embodiments 38-39, wherein the sheath is removed from at least a portion of the vasculature after the catheter and the stabilizer are advanced through the stabilizer.
41. The method of any one of embodiments 38-40, wherein the catheter and the stabilizer are coupled such that they advance through the vasculature at the same time.
42. The method of any one of embodiments 38-41, wherein the expanding the stabilizer radially outward comprises extending one or more legs of the stabilizer radially outward.
43. The method of any one of embodiments 38-42, wherein the expanding the stabilizer radially outward comprises extending one or more skis of the stabilizer radially outward.
44. The method of any one of embodiments 38-43, wherein the expanding the stabilizer radially outward comprises extending one or more fins of the stabilizer radially outward.
45. The method of any one of embodiments 38-44, wherein the expanding the stabilizer radially outward comprises inflating one or more inflatable members of the stabilizer radially outward.
46. The method of any one of embodiments 38-45, wherein the expanding the stabilizer radially outward comprises extending a shape-set wire from a channel of the stabilizer.
47. The method of any one of embodiments 38-46, wherein the expanding the stabilizer radially outward comprises extending a shape-set sheet from a channel of the stabilizer.
48. The method of any one of embodiments 38-47, further comprising implanting a valve repair or replacement device from the catheter to treat a native heart valve.
49. The method of embodiment 48, wherein the valve repair or replacement device comprises at least one of a valve repair device, a valve replacement device, an annuloplasty system, an annuloplasty structure, an annuloplasty ring, and a docking station.
50. The method of any one of embodiments 38-47, further comprising implanting an annuloplasty system or annuloplasty structure from the catheter to treat a native heart valve.
51. The method of embodiment 50, wherein implanting an annuloplasty system or annuloplasty structure from the catheter to treat a native heart valve comprises flexing or steering the distal end of the catheter to multiple locations around an annulus of the native heart valve and anchoring multiple anchors into tissue of the annulus at the multiple locations.
52. The method of embodiment 51, further comprising tensioning a tensioning member or tether of the annuloplasty system or annuloplasty structure to pull the multiple anchor and thereby the tissue of the multiple locations closer together to reshape the annulus.
53. A valve repair system for repairing a valve of a patient, the valve repair system comprising:
   a stabilizer coupled with the catheter at a position that is proximal to the steerable distal end;
   wherein the stabilizer is movable radially outward relative to the catheter to stabilize the catheter during flexing of the distal end of the steerable catheter; and
      a valve repair or replacement device coupled to the delivery catheter.
54. The valve repair system of embodiment 53, wherein the valve repair or replacement device comprises an annuloplasty ring, a valve repair device, an implantable valve replacement device, or a docking stations for valve a replacement device.

## Claims

1. A catheter stabilization system comprising:
a catheter (820) having a steerable distal end (822);
a stabilizer (830) coupled or coupleable to the catheter (820) at a position that is proximal to the steerable distal end (822);
wherein the stabilizer (830) is movable radially outward relative to the catheter (820) to stabilize the catheter (820) during flexing of the steerable distal end (822) of the catheter (820);
wherein the stabilizer (830) is configured to expand and engage vasculature to stabilize the catheter (820); and
wherein in a non-expanded state, the stabilizer (830) is flush with the catheter (820).

2. The catheter stabilization system of claim 1, wherein the catheter (820) comprises one or more of a guide sheath, a steerable catheter (820), and an implant catheter (820).

3. The catheter stabilization system of claim 1, wherein the stabilizer (830) comprises one or more legs (232) coupled with the catheter (820).

4. The catheter stabilization system of claim 3, wherein the one or more legs (232) move radially outward from the catheter (820) to stabilize the catheter (820).

5. The catheter stabilization system of claim 1, wherein the stabilizer (830) comprises one or more skis (332) coupled with the catheter (820).

6. The catheter stabilization system of claim 5, wherein the one or more skis (332) can move radially outward.

7. The catheter stabilization system of claim 1, wherein the stabilizer (830) comprises one or more fins (432) coupled with the catheter (820).

8. The catheter stabilization system of claim 7, wherein the one or more fins (432) move radially outward to stabilize the catheter (820).

9. The catheter stabilization system of claim 1, wherein the stabilizer (830) comprises one or more inflatable members (532) coupled with the catheter (820).

10. The catheter stabilization system of claim 9, wherein the one or more inflatable members (532) expand radially outward upon inflation to stabilize the catheter (820).

11. The catheter stabilization system of claim 1, wherein the stabilizer (830) comprises a channel (740) and a shape-set wire (750) within the channel (740).

12. The catheter stabilization system of claim 11, wherein the shape-set wire (750) curls back towards the catheter (820) upon being forced from the channel (740).

13. The catheter stabilization system of claim 1, wherein the stabilizer (830) comprises a shape-set mesh material (840); preferably wherein the shape-set mesh material (840) curls back towards the catheter (820) upon being pushed out of the catheter (820).

14. The catheter stabilization system of claim 1, further comprising a valve repair or replacement device configured to be delivered to a native heart valve by the catheter (820).

15. The catheter stabilization system of claim 14, wherein the valve repair or replacement device comprises at least one of a valve repair device, a valve replacement device, an annuloplasty system, an annuloplasty structure, an annuloplasty ring, and a docking station.
